(19) Europäisches Patentamt
European Patent Office
Office européen des brevets

(11) Veröffentlichungsnummer: **0 301 391 B1**

(12) # EUROPÄISCHE PATENTSCHRIFT

(45) Veröffentlichungstag der Patentschrift: **17.02.93**

(51) Int. Cl.[5]: **C12P 13/04**, C12P 41/00

(21) Anmeldenummer: **88111636.2**

(22) Anmeldetag: **20.07.88**

(54) **Verbessertes Verfahren zur enzymatischen Herstellung von L-2-Amino-4-methylphosphino-buttersäure.**

(30) Priorität: **25.07.87 DE 3724722**

(43) Veröffentlichungstag der Anmeldung:
**01.02.89 Patentblatt 89/05**

(45) Bekanntmachung des Hinweises auf die Patenterteilung:
**17.02.93 Patentblatt 93/07**

(84) Benannte Vertragsstaaten:
**AT BE CH DE ES FR GB GR IT LI NL SE**

(56) Entgegenhaltungen:
**EP-A- 0 034 293**
**EP-A- 0 054 897**
**FR-A- 2 438 054**
**GB-A- 1 369 462**

(73) Patentinhaber: **HOECHST AKTIENGESELL-SCHAFT**
**Postfach 80 03 20**
**W-6230 Frankfurt am Main 80(DE)**

(72) Erfinder: **Wullbrandt, Dieter, Dr.**
**Bienerstrasse 29**
**W-6238 Hofheim am Taunus(DE)**
Erfinder: **Keller, Reinhold, Dr.**
**Wiesenweg 5**
**W-6232 Bad Soden am Taunus(DE)**

EP 0 301 391 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

L-2-Amino-4-methylphosphinobuttersäure (im folgenden als L-Phosphinothricin oder L-PTC bezeichnet) oder deren Salze mit organischen oder anorganischen Basen oder Säuren sind -wie auch aus DOS 2939269 bekannt geworden ist - die wirksame Komponente der chemisch leicht zugänglichen Racemate. L-PTC besitzt gemäß DOS 27 17 440 eine sehr gute und breite herbizide Wirksamkeit gegenüber zahlreichen monokotylen und dikotylen, einjährigen und mehrjährigen Unkräutern. Da L-PTC und seine oben angeführten Derivate im Vergleich zu den Racematen etwa doppelt so stark wirksam ist, war es wünschenswert, ein Verfahren zu entwickeln, mit dem es möglich ist, L-PTC auf einfache Weise in größeren Mengen zugänglich zu machen.

L-PTC kann durch saure Hydrolyse (JA-OS 73-85538) oder enzymatischen Abbau (JA-OS 74-31890) von L-PTC-alanylalanin, einem literaturbekannten mikrobiell gewonnenen Antibiotikum erhalten werden.

In DOS 29 39 269 ist ferner ein Verfahren beschrieben, bei dem N-Acyl-(insbesondere N-Acetyl)-L-PTC mittels mikrobieller Acylasen, die in Form ganzer Zellen oder Zellextrakten eingesetzt werden und aus speziell gezüchteten Stämmen der Gattung Pseudomonas, Streptomyces oder Aspergillus stammen, schneller gespalten wird als das entsprechende D-Derivat. Das nach der Aufarbeitung isolierte PTC hat nur einen maximalen spez. Drehwert $[\alpha]_D^{22}$ von 23° (c = 1, 1N HCl), was einer optischen Reinheit von nur etwa 75 % entspricht.

Aus der Deutschen Patentschrift 30 48 612 ist bekannt, daß Penicillin-G-Acylase, insbesondere aus E. coli ATCC 11105, die N-Aracetylderivate des racemischen Phosphinothricins mit vergleichsweise hoher Ausbeute spalten, in freier wie auch in immobilisierter Form.

Überraschend wurde nun gefunden, daß mit Hilfe von immobilisierten E. coli Zellen das Verfahren aufgrund der besseren Standzeiten und geringerer Aktivitätsverluste wesentlich verbessert werden kann.

Die Erfindung betrifft somit ein Verfahren zur enzymatischen Trennung von D,L-2-Amino-4-methylphosphinobuttersäure, das dadurch gekennzeichnet ist, daß man deren N-Phenacetylderivate der Formel I

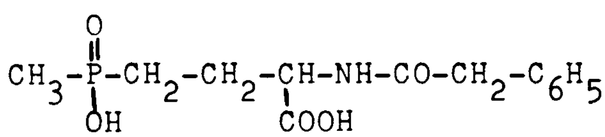

(I)

in wäßrigem oder wäßrig-organischem Medium mit an/in Chitosan immobilisierten Zellen von E. coli ATCC 11105 inkubiert wird.

Die Erfindung wird im folgenden detailliert beschrieben, insbesondere in ihren bevorzugten Ausführungsformen. Ferner wird die Erfindung in den Ansprüchen definiert.

Die Zellen von E. coli ATCC 11105 werden in herkömmlichen, dem Fachmann an sich bekannten Nährmedien gezüchtet. Zur Immobilisierung an Chitosan werden sie von dem Nährmedium abgetrennt und nach bekannten Methoden an Chitosan fixiert, bevorzugt nach dem Verfahren der Deutschen Patentschriften 28 35 875, 30 05 632 oder 30 03 633. Die Inkubation des Substrats mit den an Chitosan immobilisierten E. coli-Zellen kann im Batch- oder kontinuierlichen Säulenverfahren durchgeführt werden. Um das Verfahren so wirtschaftlich wie möglich zu gestalten, wird bevorzugt kontinuierlich gearbeitet, d. h. die Substratlösung wird kontinuierlich bis zur vollständigen Deacylierung des N-Phenacetyl-L-Phosphinothricins über das fixierte Enzym als stationäre Phase, zweckmäßigerweise in einer Säule, geleitet.

Die wäßrige Substratlösung enthält das racemische Phosphinothricinderivat in Konzentrationen von ca. 0,1 bis 30 %, bevorzugt 7 bis 15 %. Die Reaktionstemperatur liegt zwischen 20 und 45°C, bevorzugt zwischen 30 und 40°C. Ausreichende Reaktivität der Zellen erhält man bei einem pH-Wert von 3 bis 9, bevorzugt bei 6 bis 8,5. Die Zugabe von organischen Lösungsmitteln, wie beispielsweise Essigsäurebutylester, tert.-Butylmethylether oder Methylisobutylketon, zu der wäßrigen Reaktionslösung bis zur Sättigung bzw. ein Waschen des Immobilisats in regelmäßigen Abständen mit diesen Lösungsmitteln wirkt sich vorteilhaft auf dessen Standzeiten aus.

Verlauf und Ende der Reaktion können mit Hilfe polarimetrischer Methoden oder der literaturbekannten quantitativen Analyse der gebildeten freien Aminosäuren durch Umsetzung mit Ninhydrin und photometrische Gehaltsbestimmung verfolgt werden. Bevorzugt wird jedoch die Freisetzung der Phenylessigsäure mit Hilfe der HPLC verfolgt.

Das in hoher Ausbeute erhaltene L-PTC hat einen Drehwert $[\alpha]_D^{22}$ = +28,5°(c = 1 in 1N HCl), was einer optischen Reinheit von mindestens 90 % entspricht.

Überraschend wurde gefunden, daß die Wirtschaftlichkeit der biologischen Racematspaltung nach dem erfindungsgemäßen Verfahren unter Verwendung von E. coli ATCC 11105-Zellen wesentlich verbessert werden kann, trotz der Freisetzung von Phenylessigsäure während des Reaktionsverlaufs, aufgrund eines signifikant geringeren Aktivitätsverlustes und besserer Standzeiten des Biokatalysators im Vergleich zu immobilisierten Penicillinacylasen.

Die im folgenden aufgeführten Beispiele dienen der weiteren Erläuterung der Erfindung.

**Beispiele**

Penicillin-G-Acylase wird gemäß DOS 27 32 301, Beispiel 1 oder 2, (Biokatalysator 1) oder gemäß DOS 3 344 912, Beispiel 7, (Biokatalysator 2) fixiert. E. coli ATCC 11105 wird gemäß DE 30 05 632, Beispiel 1, (Biokatalysator 3) immobilisiert. Biokatalysator 1 und 2 haben eine Aktivität von ca. 170 Units/g Träger (feucht) x min, und Biokatalysator 3 hat eine Aktivität von 25 Units/ml Träger x min, jeweils gegenüber der Hydrolyse von Penicillin-G-Kaliumsalz (1 %ige wäßrige Lösung) bei 37° C.

**Beispiel 1**

Eine 10 %ige Phenacetylphosphinothricin-di-Natrium- oder -di-Ammoniumsalzlösung wird bei einem pH-Wert von 7,7 auf 35° C temperiert und anschließend mit einer Flußrate von größer 20 l/h über eine mit 10 g Biokatalysator 1 oder 2 gefüllte Glassäule gepumpt. Der pH-Wert wird mit 0,1 normaler Natronlauge oder 0,1 normaler Ammoniaklösung auf 7,7 nachreguliert. Die Substratlösung und der Produktstrom wird mit 35 ml pro Stunde (je nach angestrebtem Umsatz) zu- bzw. abgeführt.

Die Aktivität des Biokatalysators wird gegenüber der Hydrolyse einer 1 %igen wäßrigen Penicillin-G-Kaliumsalz-Lösung bei einer Temperatur von 37° C direkt bzw. nach Regenerierung durch Waschen mit Butylacetatlösung bestimmt.

**Beispiel 2**

Entsprechend Beispiel 1 unter Verwendung von 30 ml fixierter E. coli-Zellen (Biokatalysator 3) wird die enzymatische Hydrolyse der 10 %igen Phenacetylphosphinothricin-Lösung im Wirbelbett bei pH 8,1 durchgeführt. Die Enzymaktivität bezüglich der Spaltung von Penicillin-G-Kaliumsalz wurde gemäß Beispiel 1 bestimmt.

**Beispiel 3**

In einem 1 l-Rührreaktor mit pH-Steuerung werden 480 ml 10 %ige Phenacetylphosphinothricin-Lösung bei pH 7,7 auf 35° C temperiert. Durch Zugabe von 10 g feuchtem Biokatalysator 1 wird die Reaktion gestartet und der pHWert durch Zugabe von verdünnter Ammoniaklösung nachreguliert. Nach 24 h wird die Reaktionslösung vom Biokatalysator abgetrennt und die Reaktion durch Zugabe neuer Substratlösung gestartet.

Die Ergebnisse der Beispiele werden in der nachfolgenden Tabelle aufgeführt.

| Beispiel | Träger | Reaktionszeit d | Aktivitätsverlust % |
|---|---|---|---|
| 1 | 1 | 7 | 16 |
| | 2 | 7 | 17 |
| | 1 | 18 | 29 |
| | 1 | 30 | 50 |
| 2 | 3 | 10 | 0 |
| | 3 | 23 | 3 |
| 3 | 1 | 7 | 16 |

**Beispiel 4:**

**Verfahrensanalytik, Umsatzbestimmung:**

HPLC
Säule: RP 8
Flow : 2 ml/min
Druck: 160 - 180 bar
Solvens: Wasser-Methanol 50 : 50 (v/v) pH 2,1
Detektion Phenylessigsäure: UV 206 nm

**Patentansprüche**

1. Verfahren zur enzymatischen Trennung von D,L-2-Amino-4-methylphosphinobuttersäure, dadurch gekennzeichnet, daß deren Phenacetylderivate der Formel I

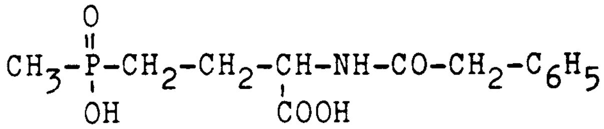

(I)

in wäßrigem oder wäßrig-organischem Medium mit an/in Chitosan immobilisierten Zellen von E. coli ATCC 11105 inkubiert wird.

2. Verfahren nach Anspruch 1, dadurch gekennzeichnet, daß das wäßrige Medium mit Essigsäurebutylester, tert.-Butylmethylether oder Methylisobutylketon gesättigt ist.

3. Verfahren nach Anspruch 1 oder 2, dadurch gekennzeichnet, daß bei 20 bis 45 ° C inkubiert wird.

4. Verfahren nach Anspruch 3, dadurch gekennzeichnet, daß bei 30 bis 40 ° C inkubiert wird.

5. Verfahren nach einem oder mehreren der Ansprüche 1 bis 4, dadurch gekennzeichnet, daß bei einem pH-Wert von 3 bis 9 inkubiert wird.

6. Verfahren nach Anspruch 5, dadurch gekennzeichnet, daß bei einem pH-Wert von 6 bis 8,5 inkubiert wird.

## Claims

1. A process for the enzymatic resolution of D,L-2-amino-4-methylphosphinobutyric acid, which comprises incubating N-phenacetyl derivatives thereof of the formula I

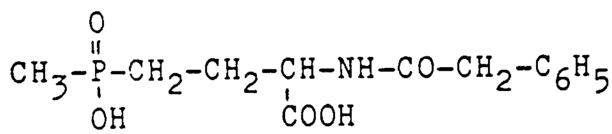

(I)

in an aqueous or aqueous-organic medium with E. coli ATCC 11105 cells immobilized on/in chitosan.

2. The process as claimed in claim 1, where in the aqueous medium is saturated with butyl acetate, tert.-butyl methyl ether or methyl isobutyl ketone.

3. The process as claimed in claim 1 or 2, wherein the incubation is carried out at 20 to 45 ° C.

4. The process as claimed in claim 3, wherein the incubation is carried out at 30 to 40 ° C.

5. The process as claimed in one or more of claims 1 to 4 , where in the incubation is carried out at a pH from 3 to 9.

6. The process as claimed in claim 5, wherein the incubation is carried out at a pH from 6 to 8.5.

## Revendications

1. Procédé pour isoler, par voie enzymatique, l'acide D,L-2-amino-4-méthylphosphinobutyrique, caractérisé en ce qu'on incube des dérivés phénacétyle de celui-ci, de formule I

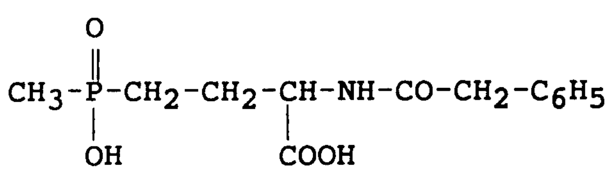

(I)

en milieu aqueux ou organo-aqueux, avec des cellules d'E. coli ATCC 11105, immobilisées sur/dans du chitosane.

2. Procédé selon la revendication 1, caractérisé en ce que le milieu aqueux est saturé avec de l'acétate de butyle, de l'éther tert.-butylméthylique ou de la méthylisobutylcétone.

3. Procédé selon la revendication 1 ou 2, caractérisé en ce qu'on effectue l'incubation à une température allant de 20 à 45 ° C.

4. Procédé selon la revendication 3, caractérisé en ce qu'on effectue l'incubation à une température allant de 30 à 40 ° C.

5. Procédé selon une ou plusieurs des revendications 1 à 4, caractérisé en ce qu'on effectue l'incubation à un pH allant de 3 à 9.

6. Procédé selon la revendication 5, caractérisé en ce qu'on effectue l'incubation à un pH allant de 6 à 8,5.